(19) Europäisches Patentamt
European Patent Office
Office européen des brevets

(11) EP 0 675 105 B1

(12) FASCICULE DE BREVET EUROPEEN

(45) Date de publication et mention de la délivrance du brevet:
**23.12.1998 Bulletin 1998/52**

(51) Int Cl.[6]: **C07C 233/69**, C07C 233/22, A61K 49/04

(21) Numéro de dépôt: **95400611.0**

(22) Date de dépôt: **20.03.1995**

# (54) Composés polyiodés, procédé de préparation et composition de diagnostic

Polyiodierte Verbindungen, Verfahren zu deren Herstellung und diagnostische Zusammensetzung

Polyiodinated compounds, process for their preparation and diagnostic composition

(84) Etats contractants désignés:
**AT BE CH DE DK ES FR GB GR IE IT LI LU NL PT SE**

(30) Priorité: **22.03.1994 FR 9403356**

(43) Date de publication de la demande:
**04.10.1995 Bulletin 1995/40**

(73) Titulaire: **GUERBET S.A.**
**F-93430 Villepinte (FR)**

(72) Inventeurs:
- **Petta, Myriam**
**F-93600 Aulnay-sous-Bois (FR)**
- **Meyer, Dominique**
**F-94100 Saint-Maur (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 558 395**
**WO-A-92/08691**
**GB-A- 881 510**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 675 105 B1

Printed by Jouve, 75001 PARIS (FR)

## Description

La présente invention concerne des composés polyiodés, leur procédé de préparation et leur utilisation comme agent de contraste lors de radiographies par rayons X chez l'homme.

Ces composés sont des dérivés du benzène, substitué par 4 ou 5 atomes d'iode.

Des dérivés benzéniques tétraiodés ou pentaiodés ont déjà été cités parmi d'autres agents de contraste, notamment dans les brevets FR 828 486, US 3 042 715, CH 615 344 et GB 881 510. Tous ces dérivés comportaient au moins un groupe carboxylique ou amino, de telle sorte que certains de leurs sels présentaient la solubilité aqueuse requise pour leur administration par injection. Néanmoins, on sait que l'osmolalité d'une solution d'agent de contraste, qui augmente avec la concentration en ions du milieu, peut être à l'origine d'effets secondaires lors de l'injection lorsqu'elle est nettement supérieure à celle du milieu biologique.

Il était donc souhaitable de trouver des composés à fort pourcentage pondéral en iode, de façon à pouvoir diminuer la dose d'agent de contraste injectée au sujet, solubles dans l'eau de façon à ce que le volume total injecté reste compris dans les limites habituelles, de 10 à 300 ml suivant l'organe à visualiser, et dont les solutions présentent une osmolalité inférieure à celle des composés mentionnés précédemment ainsi qu'une viscosité modérée.

Les composés selon l'invention sont de formule :

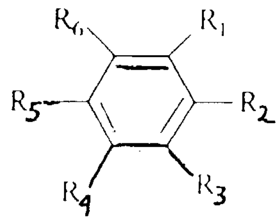

(I)

dans laquelle $R_1$, $R_2$, $R_3$, $R_4$, $R_5$, $R_6$ sont choisis indépendamment parmi l'atome d'iode et les groupes :

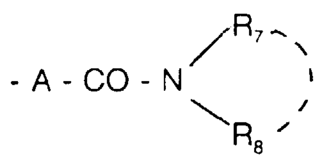

;

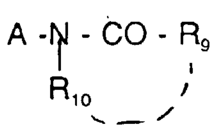

;

et

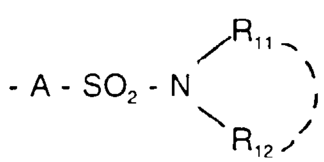

;

dans lesquels

A représente rien ou ($C_1$-$C_4$) alkylène, éventuellement hydroxylé;

$R_7$, $R_8$, $R_9$, $R_{10}$, $R_{11}$ et $R_{12}$, identiques ou différents, représentent H, ($C_1$-$C_{10}$) alkyle, éventuellement hydroxylé ou portant un ou des groupes ($C_1$-$C_3$)alkoxy, éventuellement hydroxylés ou, avec l'atome d'azote auquel ils sont attachés, $R_7$ et $R_8$, $R_9$ et $R_{10}$ ou $R_{11}$ et $R_{12}$ peuvent former indépendamment un hétérocycle saturé de 5 à 8 sommets, éventuellement hydroxylé,

étant entendu qu'au moins 4 groupes parmi $R_1$, $R_2$, $R_3$, $R_4$, $R_5$ et $R_6$ représentent l'atome d'iode et que les composés de formule I contiennent de 10 à 24 groupes hydroxyle.

Les groupes alkyle, alkoxy, alkylène sont linéaires ou ramifiés.

On préfère les composés de formule I tétraiodés dans lesquels les deux autres substituants représentent :

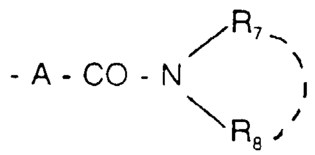

ou

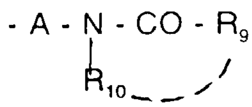

A étant $CH_2$ ou rien,
et particulièrement ceux pour lesquels A n'est rien, $R_2 = R_3 = R_5 = R_6 = I$ et $R_1$ et $R_4$ représentent indépendamment un groupe $-CO-NR_7R_8$ hydroxylé ou un groupe $-N(R_{10})-CO-R_9$ hydroxylé.

Avantageusement, les composés de formule I portent de 12 à 20 groupes hydroxyle, de préférence de 12 à 16 groupes hydroxyle et 2 groupes au moins parmi $R_7$, $R_8$, $R_9$ et $R_{10}$ représentent
$-(CH_2)_m - (CHOH)_n - CH_2OH$ et m = 1 ou 2, n = 0 à 4;

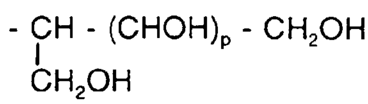

et p = 0 à 3 ; ou
$-C(CH_2OH)_3$.

Les composés tétraiodés qui comportent deux groupes amides en para, identiques ou non, de formule :

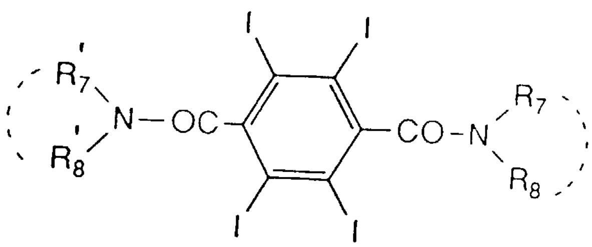 (II)

dans laquelle $R_7$, $R_8$, $R'_7$ et $R'_8$, identiques ou différents, ont la même signification que $R_7$ et $R_8$ dans la formule 1, sont particulièrement préférés, notamment pour leur tolérance et leur facilité de synthèse.

Lorsque les deux groupes amides sont différents, il est préférable que chacun comporte au moins deux hydroxyles, notamment pour une répartition plus équilibrée de l'hydrophilie.

Lorsque les deux groupes amides sont identiques, on préfère que le composé de formule II comporte de 12 à 20 groupes hydroxyle et que $R_7$ et $R_8$ soient choisis parmi les groupes
$- CH_2 - (CHOH)_n - CH_2OH$ avec n = 0 à 4, et

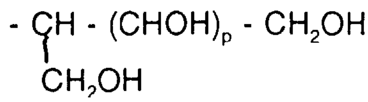

avec p = 1 ou 3

et mieux, qu'ils soient choisis parmi les groupes $- CH_2 - (CHOH)_n - CH_2OH$ avec n = 0 à 3, ledit composé de formule II comportant alors de 12 à 16 groupes hydroxyle, notamment pour avoir des produits solubles peu visqueux.

La préparation de certains composés de formule I ou II de l'invention met en oeuvre les aminoalcools de formule $HNR_7R_8$, $HNR'_7R'_8$ ou $HNR_{11}R_{12}$.

De nombreux aminoalcools hydroxylés sont connus et même commercialisés.

Parmi ces derniers, on peut citer :

(i) 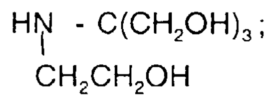

(ii) 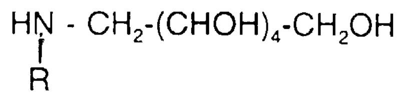

avec R = $CH_3$, $CH_2CH_2OH$; ou $CH_2(CHOH)_4CH_2OH$

(iii) 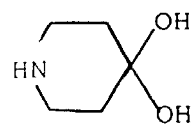

et (iv) la glucosamine.

D'autres aminoalcools sont décrits dans la littérature; ils sont préparés notamment par monosubstitution d'aminoalcools primaires ou par disubstitution de la benzylamine suivie d'une débenzylation, dans des conditions classiques, notamment par action de $H_2$.

Parmi les aminoalcools primaires commerciaux, on peut citer :

$H_2N- CH_2 - CHOH - CH_3$, $H_2N - CH_2 - CHOH - CH_2OH$, $H_2N - (CH_2)_3 - CH_2OH$, $H_2N - CH(CH_2OH)_2$, $H_2N - C(CH_2OH)_3$ et

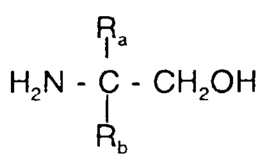

avec

$R_a = H$, $R_b = CH_3$ ou $C_2H_5$ ; ou bien
$R_a = CH_3$, $R_b = CH_2OH$.

Parmi les aminoalcools connus préférés, on peut citer :

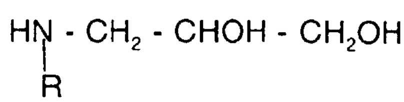

avec R = $CH_3$, décrit dans WO 89/10752
avec R = $CH_2CH_2OH$, décrit dans US 5,075,502
avec R = $C(CH_2OH)_3$ décrit dans J. Amer. Chem. Soc., 66, 881 (1944);

- et ceux décrits dans EP-A-558 395, qui sont :

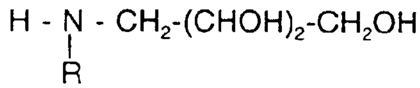

avec R = $CH_3$, $CH_2$- $CH_2$- OH, $CH_2$ - CHOH - $CH_2OH$, $CH_2$-$(CHOH)_2$-$CH_2$ OH et $CH(CH_2OH)_2$
et

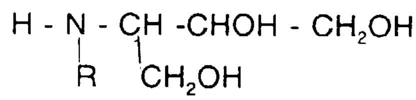

avec R = H, $CH_3$, $CH_2CH_2OH$, $CH_2$-CHOH-$CH_2OH$ ou $CH(CH_2OH)_2$
et

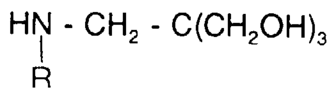

avec R = $CH_3$, $CH_2CH_2OH$ et $CH_2$ - CHOH - $CH_2OH$
- ou encore ceux décrits dans EP-A-25083 qui sont :

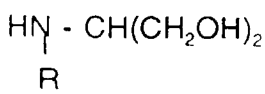

avec R = $CH_3$, $C_2H_5$, $CH_2CH_2OH$, $CH_2$ - CHOH - $CH_2OH$ et $CH(CH_2OH)_2$.

Parmi les aminoalcools hétérocycliques, on peut citer :

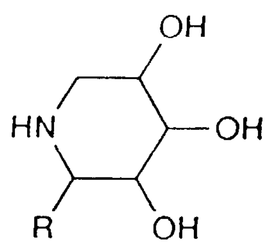

R = H, OH
décrit dans Chem. Ber., 692, p. 200-214.

Il est entendu que les amides selon l'invention ne sauraient être limités à ceux préparés à partir des aminoalcools précédemment cités ou déjà connus.

Les composés de formule I pentaiodés dans lesquels le dernier substituant est

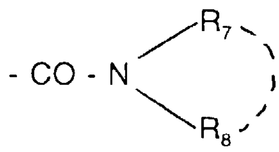

peuvent être préparés par action, sur l'aminoalcool, du chlorure de l'acide pentaiodobenzoïque obtenu par action du chlorure de thionyle sur l'acide pentaiodobenzoïque décrit dans J. Org. Chem. 49 (17) 3051-3053 (1984).

Les composés de formule I tétraiodés comportant deux groupes amido

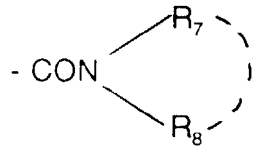

peuvent être préparés à partir des acides tétraiodoisophtaliques et tétraiodotéréphtaliques, obtenus par iodation des diacides correspondants comme décrit dans FR-A-828 486.

En outre, les dérivés isophtaliques peuvent être obtenus par une réaction de Sandmeyer avec KI sur le dérivé diazoïque de l'acide triiodoaminoisophtalique, connu.

Des conditions d'amidification des acides benzoïques comportant en ortho deux atomes d'iode sont connues de l'homme du métier et décrites dans de nombreuses publications et brevets.

On peut citer par exemple FR-A-2 354 316, FR-A-2 293 919, EP-A-15867, EP-A-32388 ou EP-A-233249.

Lorsque les deux groupes amides sont différents, on peut effectuer l'amidification comme décrit dans FR-A-2 023 741 par action d'un premier aminoalcool introduit par petites portions sur un dérivé activé - sous forme halogénure d'acide, ester ou anhydride - du diacide, puis action d'un second aminoalcool sur le monoamide monoacide activé.

L'amidification des groupes - A - COOH dans lesquels A est différent de rien peut être effectuée de façon classique.

Les composés de formule I tétraiodés dans lesquels

$$R_2 = R_3 = R_5 = R_6 = I;$$

$R_1$ = 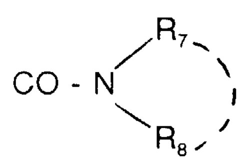 ;

et

$R_4$ = 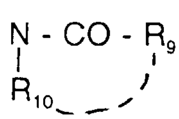

peuvent être préparés à partir du p-nitrotoluène par la méthode suivante:

1/ iodation du p-nitrotoluène pour obtenir le 2,6-diiodo-4-nitrotoluène ;

2/ action du N-bromosuccinimide pour obtenir le composé de formule :

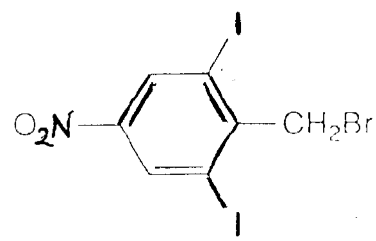

3/ oxydations pour obtenir la 2,6-diiodo-4-nitrobenzaldéhyde, puis l'acide 2,6-diiodo-4-nitrobenzoïque ;

4/ réaction d'un dérivé activé de cet acide avec un aminoalcool pour obtenir

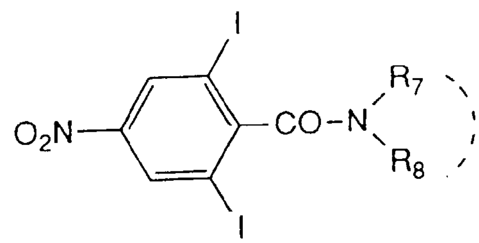

5/ réduction catalytique du groupe nitro en amino ;

6/ iodation du composé obtenu pour obtenir un dérivé tétraiodé ;

7/ acylation de la fonction amine avec un dérivé activé de l'acide $R_9$ - COOH, les hydroxyles présents ayant éventuellement été protégés ;

8/ substitution du groupe amide secondaire formé par action d'un composé de formule $R_{10}$ - X" dans laquelle X" représente un halogène, et particulièrement Cl ou Br ou un groupe $(C_1$-$C_4)$alkylsulfonyle tel que le groupe méthylsulfonyle ou $(C_6$-$C_{10})$ arylsulfonyle éventuellement substitué par $(C_1$-$C_4)$alkyle tel que le groupe p-toluènesulfonyle;

9/ le cas échéant, déprotection des groupes hydroxyles.

La déprotection des groupes hydroxyles peut être effectuée avant l'étape de substitution. De même, on peut inverser les étapes 7 et 8, et préparer l'aniline secondaire par action de $R_{10}$ - X" avant de faire réagir un dérivé activé de $R_9$ - COOH.

Les réactions d'iodation peuvent être effectuées, comme il est bien connu, par ICl aqueux, par $I_2$ en présence de KI et $C_2H_5NH_2$ ou $KIC_{l2}$, par $I_2$ en solution dans l'oleum, ou par un mélange $I_2/H_5IO_6$,

L'oxydation du dérivé bromé en aldéhyde peut être effectuée par action du N-oxyde de triéthylamine dans un solvant, tel que le 1,2-dichloroéthane; l'oxydation de l'aldéhyde peut être effectuée par $KMnO_4$.

Les groupes amides peuvent être obtenus de façon classique, par réaction des chlorures d'acide correspondants sur les aminoalcools appropriés, les chlorures d'acide pouvant être obtenus par action de $SOCl_2$ sur l'acide. Si nécessaire, les fonctions hydroxyles libres peuvent être protégées sous forme d'esters, acétique notamment.

Certains des aminoalcools intermédiaires sont des composés nouveaux et ils sont un autre objet de l'invention. Ces composés répondent à la formule :

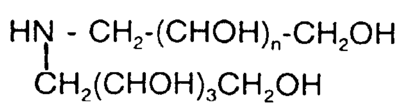

dans laquelle
n = 1 à 3,
à la formule :

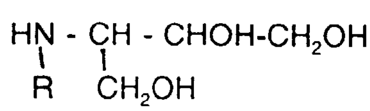

avec R = $CH_2(CHOH)_pCH_2OH$ avec p = 2, 3,
et à la formule :

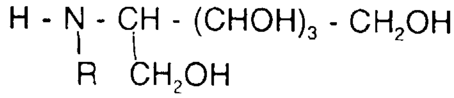

dans laquelle R = $CH_2(CHOH)_p$-$CH_2OH$ avec p = 1 à 3.

Ces composés peuvent être préparés par amination réductrice de xylose, érythrose ou fructose, avec un aminoalcool ou l'ammoniac, notamment par l'hydrogène en présence d'un catalyseur d'hydrogénation tel que le charbon palladié.

L'invention concerne aussi les compositions de diagnostic utiles comme agents de contraste lors des radiographies par rayons X chez l'homme, par exemple, pour effectuer des angiographies ou des urographies ou pour opacifier différentes régions corporelles.

Ces compositions, sous forme de solutions ou éventuellement de suspensions ou d'émulsions, contenant entre 10 et 40 g de groupes iodés pour 100 ml, peuvent être administrées par voie entérale ou parentérale. Les solutions sont en général des solutions aqueuses, de préférence tamponnées; elles peuvent comprendre différents additifs, tels que des cations $Na^+$, $Ca^{2+}$, $Mg^{2+}$, des stabilisants tels que l'édétate de calcium, des agents osmolaires.

Ces compositions peuvent aussi être administrées par voie orale ou rectale, notamment pour la visualisation du système gastro-intestinal.

Les doses unitaires seront fonction de la zone à observer et du sujet traité; pour les solutions injectables, elles seront en général de 10 à 300 ml.

Dans ce qui suit, on décrit des exemples de composés de l'invention.

Les chromatographies sur couches minces ont été effectuées sur des plaques de silica-gel commercialisées par MERCK sous la référence F-254, en utilisant comme éluant, sauf mention contraire, un mélange $S_1$ de $H$-$CO_2H/CH_3$-$CO$-$C_2H_5/C_6H_5$-$CH_3$ (25/25/60 - v/v/v) en révélant sous rayons ultra-violets ou pour les aminoalcools par la ninhydrine, l'éluant étant le mélange ($C_2H_5OH/NH_4OH$ aqueux à 25 %: 7/3 - v/v).

Les spectres RMN du $^{13}C$ ont été, sauf mention contraire, effectués avec un appareil à 200 MHz dans le DMSO (diméthylsulfoxyde) comme étalon interne.

Préalablement, la préparation de divers aminoalcools, mis en oeuvre dans les exemples qui suivent, est indiquée.

A) 5-(2,3-dihydroxypropylamino)-1,2,3,4-pentanetétrol

$$(HNR_7R_8 : R_7 = CH_2\text{-}CHOH\text{-}CH_2OH,$$

$$R_8 = CH_2\text{-}(CHOH)_3\text{-}CH_2OH)$$

Un mélange de 529,5 g de D-xylose commercial et 289 g d'amino-2,3-propanediol dans 12,5 l d'éthanol, en présence de 53 g de charbon palladié à 10 % est hydrogéné sous une pression de 6 x $10^5$ Pa, à 50° C pendant 30 heures. Le catalyseur est ensuite éliminé par filtration sur célite et le filtrat concentré à sec. L'huile résiduelle dissoute dans 30 1 d'eau est passée sur 3 l de résine sous forme $H^+$, référence IRC 50 de chez Rohm et Haas.

On isole ainsi 616 g de l'aminoalcool cherché.

CCM : Rf = 0,3
RMN$^{13}C$ : δ(ppm) :
72-70 (CHOH) ; 65-63 ($CH_2OH$); 53-52 ($CH_2NH$).

B) 5-((1-hydroxyméthyl-2,3-dihydroxy)propylamino)-1,2,3,4-pentanetétrol

($HNR_7R_8$ :

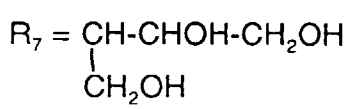

$$R_8 = CH_2\text{-}(CHOH)_3\text{-}CH_2OH)$$

6 g de 2-amino-1,3,4-butanetriol, préparé par la méthode décrite dans US 4,439,613 et 8,2 g de D-xylose commercial en solution dans 250 ml d'éthanol, avec 1 g de charbon palladié à 10 % sont hydrogénés pendant 48 heures à 50° C sous une pression de 8 x $10^5$ Pa.

Le catalyseur est alors éliminé par filtration sur célite et le filtrat concentré sous pression réduite.

Après passage sur 80 ml de résine IRC 50, on obtient 4,5 g de l'aminoalcool attendu.

CCM : Rf = 0,28
RMN$^{13}$C : δ(ppm) :
70 - 71 (CHOH); 64,63,60 ($CH_2OH$); 61 (CHNH); 50 ($CH_2NH$).

C) 5-(2,3,4,5-tétrahydroxypentylamino)-1,2,3,4-pentanetétrol

$$(HNR_7R_8 : R_7 = R_8 = CH_2(CHOH)_3 - CH_2OH)$$

Un mélange de 26 g de D-xylose, 5,5 ml de solution aqueuse de $NH_4OH$ à 25 % (p/v) dans 345 ml de méthanol est hydrogéné en présence de 2,5 g de charbon palladié à 10 %, sous une pression de 8 x $10^5$ Pa à 50° C pendant 48 heures.

Après élimination du catalyseur, la solution est concentrée sous pression réduite et l'huile résiduelle passée sur résine IRC 50 pour donner 2 g de l'aminoalcool attendu.

CCM : Rf = 0,17
RMN$^{13}$C : δ (ppm) :
72-71,5-70,5 (CHOH) ; 63 ($CH_2OH$); 51,4 ($CH_2NH$).

D) 5-(2,3-dihydroxypropylamino)-1,2,3,4,6-hexanepentol

$$(HNR_7R_8 : \quad R_7 = CH_2\text{-}CHOH\text{-}CH_2OH$$
$$R_8 = \underset{\displaystyle CH_2OH}{\underset{|}{CH}}\text{-}(CHOH)_3\text{-}CH_2OH)$$

Un mélange de 1,9 g d'amino-2,3-propanediol, 4,5 g de fructose commercial dans 150 ml de méthanol est hydrogéné pendant 6 heures sous une pression de 14 x $10^5$ Pa à 80° C, en présence de 2 g de charbon palladié.

Après filtration sur célite pour éliminer le catalyseur, la solution est concentrée; l'huile résiduelle dissoute dans 10 ml d'eau est chromatographiée sur une résine sous forme $H^+$ (IRN 77) en éluant avec une solution aqueuse de $NH_4OH$, de concentration croissante.

On isole finalement 2,1 g du produit attendu sous forme d'huile.

CCM : Rf = 0,25
RMN$^{13}$C : 67-74 (CHOH) ; 64 ($CH_2OH$) ; 62 ($\underline{C}H\text{-}CH_2OH$); 50 ($\underline{C}H_2CH$).

En appliquant le mode opératoire précédent, on prépare le 4-((1-hydroxyméthyl-2,3-dihydroxy)propylamino)-1,2,3-butanetriol à partir de l'érythrulose et de $H_2N\text{-}CH_2(CHOH)_2\text{-}CH_2OH$.

$$(HNR_7R_8 : \quad R_7 = \underset{\displaystyle CH_2OH}{\underset{|}{CH}} - CHOH - CH_2OH$$
$$R_8 = CH_2\text{-}(CHOH)_2 - CH_2OH)$$

CCM : Rf = 0,55

E) 5-(2,3,4-trihydroxybutylamino)-1,2,3,4-pentanetétrol

$(HNR_7R_8 : R_7 = CH_2 - (CHOH)_2 - CH_2OH$

$R_8 = CH_2 - (CHOH)_3 - CH_2OH)$

i) butène-3,4-diol : $H_2C = CH - CHOH - CH_2OH$

250 g de cis-2-butène-1,4-diol commercial sont dissous dans 1500 ml d'eau contenant 15 ml de solution aqueuse de HCl 10 N et 3 g de CuCl. Après 16 heures à la température de reflux, le pH du mélange, revenu à température ambiante, est amené à 7 par addition d'une solution aqueuse de NaOH 2N, puis le milieu réactionnel est filtré sur 500 g de célite (silice diatomée).

L'eau est alors éliminée sous pression réduite et le résidu huileux distillé pour donner 86 g du produit attendu.

$E_b$ = 70° C sous 200 Pa
CCM ($CH_2Cl_2CH_3OH$ : 90/10 : v/v)
Rf = 0,7

ii) N,N-dibenzylamino-2,3,4-butanetriol

$((C_6H_5-CH_2)_2 N - CH_2 - (CHOH)_2 - CH_2OH)$

31,4 g de l'huile obtenue, 1g de $H_2WO_4$ et 1,4 g de $CH_3COONa$ sont introduits dans 120 ml d'eau, puis 20,5 ml de $H_2O_2$ à 50% sont ajoutés, goutte à goutte.

Après 6 heures d'agitation, on introduit lentement 23,4 g de dibenzylamine en solution dans 300 ml d'éthanol. Le mélange est maintenu 18 heures sous agitation à 40° C avant d'être concentré sous pression réduite. Le résidu cristallise dans l'éther éthylique. On isole ainsi 30 g du produit attendu.

F = 95° C.

iii) 1-amino-2,3,4-butanetriol

$(H_2N - CH_2 - (CHOH)_2 - CH_2OH)$

17 g du produit précédent en solution dans 400 ml d'éthanol sont hydrogénés à 50° C sous une pression de 7 x $10^5$ Pa en présence de 3,5 g de charbon palladié (10 %) pendant 7 heures. Après élimination du catalyseur et concentration sous pression réduite, on chromatographie l'huile résiduelle, en éluant avec un mélange de $CH_3OH/NH_4OH$ aqueux à 25 % (8/2, v/v) sur silice pour obtenir 4,5 g de l'aminoalcool pur.

RMN$^{13}$C : δ(ppm) : 72 (CHOH) ; 62 ($CH_2OH$) ; 45 ($CH_2N$)

iv) 1,4 g de l'aminoalcool primaire isolé précédemment et 1,93 g de xylose en solution dans 70 ml de méthanol sont hydrogénés sous pression de 12 x $10^5$ Pa, à 50° C pendant 6 heures en présence de 0,7 g de palladium sur charbon (10 %). Aprés élimination du catalyseur par filtration, on concentre à sec. Le résidu dissous dans le minimum d'eau est chromatographié sur résine IRN 77 ($H^+$) en éluant avec une solution aqueuse de $NH_4OH$ de concentration croissante.

Après élimination du solvant, on isole 1,5 g du produit attendu, huileux.

CCM : Rf = 0,3
RMN$^{13}$C : δ (ppm) : 70-75 (CHOH); 63 ($CH_2OH$); 52 ($N(CH_2)_2$).

EXEMPLE 1

N,N'-bis[(2-hydroxyéthyl)(2,3,4,5,6-pentahydroxyhexyl)]-2,4,5,6-tétraiodo-1,3-isophtalamide

(formule I : $R_2 = R_4 = R_5 = R_6 = I$ ;

$R_1 = -CO-NR_7R_8$ ;

$R_3$ = -CO-NR'$_7$R'$_8$ ;

$R_7$ = R'$_7$ = $CH_2$-$CH_2$OH; et

$R_8$ = R'$_8$ = $CH_2$-$(CHOH)_4$-$CH_2$OH).

1/ Acide tétraiodoisophtalique

2,07 g (0,03 mole) de $NaNO_2$ dissous dans 30 ml d'eau sont introduits goutte à goutte à 5° C sur 11,2 g (0,02 mole) d'acide 5-amino-2,4,6-triiodophényl-1,3-dicarboxylique dissous dans 60 ml de solution aqueuse de NaOH 1N. Le pH de la solution est amené à 2 par addition d'acide sulfurique concentré et l'agitation est maintenue 3 heures à 5° C. 9,6 g (0,06 mole) de KI en solution dans 20 ml d'eau sont alors introduits goutte à goutte dans le milieu réactionnel, préalablement amené à pH 5 par addition d'une solution aqueuse de soude. Le mélange est ensuite porté lentement à 45° C, température à laquelle il est maintenu 2 heures avant d'être ramené à température ambiante. Il est alors versé dans 500 ml d'un mélange de glace et solution aqueuse d'acide chlorhydrique 1 N. Le précipité formé est lavé par une solution aqueuse de bisulfite de sodium puis avec 150 ml de $CH_2Cl_2$ pour donner 1 1 g de cristaux beiges du produit attendu pratiquement pur (teneur en I : 97, 5 % de la théorie), rendement 82 %.

CCM (éluant $S_1$) - Rf = 0,7
RMN$^{13}$C : δ(ppm) : 168 (<u>C</u>$O_2$H); 148 (<u>C</u> - CO) ; 127, 105, 89 (C-I).

2/ Dichlorure de l'acide tétraiodoisophtalique

10,4 g (0,015 mole) du diacide, 150 ml de $SOCl_2$ et 0,2 ml de diméthylformamide (DMF) sont maintenus 8 heures à la température de reflux. $SOCl_2$ est ensuite éliminé sous pression réduite et le résidu est solidifié par grattage dans 100 ml d'éther de pétrole pour donner II g de cristaux beiges contenant 98 % de la quantité d'iode calculée et 101 % de chlore.

CCM (éluant $S_1$) - Ref = 0,1.

3/ N,N'-bis[(2-hydroxyéthyl)(2,3,4,5,6-pentahydroxyhexyl)]-2,4,5,6-tétraiodo-1,3-isophtalamide

11 g (0,015 mole) du dichlorure d'acide sont ajoutés, par portions, à une solution de 10,5 g (0,0465 mole) de 1-déoxy-1-(2-hydroxyéthylamino)-D-glucitol commercial dissous dans 120 ml de diméthylacétamide (DMAC) contenant 6,4 ml de triéthylamine. Le mélange est maintenu 24 heures sous agitation à 60° C, le sel de triéthylamine est éliminé par filtration et le filtrat est concentré sous vide. L'huile obtenue est traitée par de la résine sous forme $H^+$ commercialisée par Rohm et Haas sous la référence IRN 77 et de la résine sous forme $OH^-$ commercialisée sous la référence IRA 67, puis chromatographiée sur silice en éluant avec un mélange $CH_2Cl_2$/$CH_3OH$ (35/65 : v/v).

On isole 4,2 g de cristaux du produit attendu dont la teneur en iode est 98 % de celle prévue.

CCM ($CH_2Cl_2$/$CH_3OH$ : 40/60 : v/v) - Rf = 0,2
RMN$^{13}$C : δ (ppm) : 120 (C = O); 148 (<u>C</u> - CO); 128, 108, 92 (C - I); 69 à 73 ($CH_2$<u>C</u>HOH); 57 à 62 ($CH_2$OH); 48 et 52 N$(CH_2)_2$.

<u>EXEMPLE 2</u>

N,N'-bis[(2-hydroxyéthyl)(2,3,4,5,6-pentahydroxyhexyl)]-2,3,5,6-tétraiodo-1,4-phénylène diacétamide

(formule I : $R_2$ = $R_3$ = $R_5$ = $R_6$ = I; $R_1$ = $R_4$ = $CH_2$-CON$R_7R_8$

et $R_7$ = $CH_2$-$CH_2$OH ; $R_8$ = $CH_2$-$(CHOH)_4$-$CH_2$OH).

1/ Acide 2,3,5,6-tétraiodo-1,4-phénylène diacétique

10 g (51,5 mmoles) d'acide 1,4-phénylène diacétique commercial sont introduits lentement dans un mélange de 9,2 g (40,5 mmoles) d'acide periodique, 200 ml d'acide sulfurique concentré et 30,9 g (121,5 mmoles) d'iode, à 0° C. Après 1 heure d'agitation à cette température, le mélange est maintenu à 35° C pendant 48 heures, puis versé sur un

mélange acétate d'éthyle/glace (2 1/500 ml). Le précipité formé est lavé avec 250 ml de $CH_2Cl_2$ et de $CH_3COOC_2H_5$, pour laisser une poudre beige (96 % de la théorie en iode).

CCM (éluant $S_1$) : Rf = 0,63
RMN$^{13}$C $\delta$(ppm) : 169 ($CO_2H$); 143 (C-$CH_2$) ; 116(C-I) ; 62 ($CH_2$)

2/ Dichlorure de l'acide 2,3,5,6-tétraiodophénylène diacétique

24,5 g (0,035 mole) d'acide 2,3,5,6-tétraiodophénylène-1,4-diacétique, 1,5 ml de DMF et 590 ml de $SOCl_2$ sont maintenus pendant 8 heures au reflux. Après concentration sous pression réduite, le résidu solide est lavé avec 100 ml de $CH_2Cl_2$. Après lavage avec 250 ml d'éther de pétrole, on obtient 10,7 g du produit attendu (pourcentage en I : 98,5 % de la théorie, en Cl : 101,4 %).
CCM (éluant $S_1$) : Rf = 0,67.

3/ N,N'-bis[(2-hydroxyéthyl)(2,3,4,5,6-pentahydroxyhexyl)]-2,3,5,6-tétraiodo- 1,4-phénylène diacétamide

10,5 g (14 mmoles) du dichlorure d'acide dissous dans 90 ml de DMAC sont ajoutés goutte à goutte à une solution de 11 g (49 mmoles) de 1-déoxy-1-(2-hydroxyéthylamino)-D-glucitol, 4,9 g (49 mmoles) de triéthylamine dans 90 ml de DMAC.
Le mélange est maintenu sous agitation 2 jours à 50° C avant concentration sous pression réduite. On introduit 250 ml d'eau et le précipité apparu est isolé par filtration et lavé abondamment avec de l'eau. On obtient ainsi 14,8 g du produit attendu sous forme d'une poudre blanche qui contient 93,6 % de la quantité théorique d'iode.

CCM ($CH_2Cl_2$/$CH_3OH$ : 40/60 : v/v) : Rf = 0,2
RMN$^{13}$C $\delta$(ppm) : 168 (C=O); 145 (C-$CH_2$) 118 (C-I); 69 à 74 (CHOH); 63 ($CH_2OH$); 62 ($CH_2CO$); 59 ($CH_2OH$); 51 ($NCH_2$); 49 ($NCH_2$).

EXEMPLE 3

N,N'-bis[(2-hydroxyéthyl)(2,3,4,5,6-pentahydroxyhexyl)]-2,3,5,6-tétraio-dotéréphtalamide

(formule II : $R_7 = R'_7 = CH_2\text{-}CH_2OH$ ; $R_8 = R'_8 = CH_2\text{-}(CHOH)_4\text{-}CH_2OH$)

94,2 g (0,42 mole) de 1-deoxy-1-(2-hydroxyéthylamino)-D-glucitol commercial en solution dans 850 ml de DMAC sont introduits goutte à goutte, à 50° C, dans une solution de 98,5 g (0,14 mole) du dichlorure d'acide dans 850 ml de DMAC et 42,3 g (0,42 mole) de triéthylamine. Après 2 jours sous agitation à 60° C, le milieu est concentré sous pression réduite et l'huile obtenue purifiée par passage sur les résines IRN 77 et IRA 67, puis par chromatographie sous une pression de 52 x $10^5$ Pa sur une colonne de silice greffée (Kromasil 100 C18 - 10 µm de EKA NOBEL), avec un gradient de $H_2O$/$CH_3OH$ et un débit de 120 ml/minute.

CCM ($CH_2Cl_2$/$CH_3OH$ : 40/60 : v/v) : Rf = 0,2
RMN$^{13}$C $\delta$(ppm) : 171 (C=O); 148 (C-CO); 110 (C-I); 69 à 72 (CHOH); 63 et 58 ($CH_2OH$).

EXEMPLE 4

N,N'-tétrakis(2,3,4-trihydroxybutyl)-2,3,5,6-tétraiodotéréphtalamide

(formule II : $R_7 = R'_7 = R_8 = R'_8 = CH_2\text{-}(CHOH)_2\text{-}CH_2OH$

En utilisant le mode opératoire de l'exemple 3 à partir du même diacide et de 2,3,4-trihydroxybutylamino-1,2,3-butanetriol, on obtient le produit attendu.

CCM ($CH_2Cl_2$/$CH_3OH$ : 60/40 (v/v) : Rf = 0,2
RMN$^{13}$C $\delta$(ppm) : 171 (C=O); 148 (C-CO); 110 (CI); 66 à 73 (CHOH); 62 ($CH_2OH$); 49 à 54 ($NCH_2$).

EXEMPLE 5

N,N'-bis[(2,3-dihydroxypropyl) (2,3,4-trihydroxybutyl)]-2,3,5,6-tétraiodotéréphtalamide

(formule II : $R_7 = R'_7 = CH_2\text{-}CHOH\text{-}CH_2OH$; $R_8 = R'_8 = CH_2\text{-}(CHOH)_2\text{-}CH_2OH$

5,85 g (0,03 mole) de 5-(2,3-dihydroxypropylamino)-2,3,4-butanetriol en solution dans 75 ml de DMAC sont introduits goutte à goutte dans une solution de 7,07 g (0,01 mole) du dichlorure d'acide, de 4,17 ml (0,03 mole) de triéthylamine et de 75 ml de DMAC. Le mélange est maintenu une nuit sous agitation à 60° C puis concentré sous pression réduite. On dissout le résidu huileux dans 20 ml d'un mélange $CH_2Cl_2/C_2H_5OC_2H_5$ 1/1 (v/v) et on purifie par passage sur résine IRN 77 puis IRA 67 et chromatographie sur 40 g de silice en éluant avec un mélange $CH_2Cl_2/CH_3OH$ 1/1 (v/v).

On obtient ainsi 2,5 g du produit attendu, sous forme de poudre blanche dont le titre en iode est 97,2 % de la théorie.

CCM ($CH_2Cl_2/CH_3OH$ : 60/40 : v/v) : Rf = 0,2

RMN$^{13}$C : δ(ppm) : 174 (C=O); 150 (C-C=O); 112 (C-I) ; 70 à 76 (CHOH); 63 à 65 ($CH_2OH$); 51 à 55 ($CH_2N$).

EXEMPLE 6

N,N'-bis[(2,3-dihydroxypropyl) (2,3,4,5-tétrahydroxypentyl)]-2,3,5,6-tétraiodotéréphtalamide

(formule II : $R_7 = R'_7 = CH_2\text{-}CHOH\text{-}CH_2OH$ ;

$R_8 = R'_8 = CH_2\text{-}(CHOH)_3\text{-}CH_2OH$)

100 g (0,44 mole) de 5-(2,3 dihydroxypropylamino)-1,2,3,4-pentanetétrol en solution dans 1130 ml de DMAC sont introduits goutte à goutte, à 55° C, dans une solution de 103,5 g (0,15 mole) du dichlorure de l'acide 2,3,5,6-tétraiodotéréphtalique dans 1130 ml de DMAC contenant 57 g (0,56 mole) de triéthylamine. Après 3 jours d'agitation à cette température, le milieu réactionnel est concentré et l'huile obtenue est chromatographiée sur 1 l de résine IRM 77 puis IRA 67 avant d'être chromatographiée sous une pression de 52 x $10^5$ Pa sur une colonne de silice greffée (Kromasil 100 C18 - 10 µm de EKA NOBEL) avec un gradient de $H_2O/CH_3OH$ et un débit de 120 ml/minute pour donner 43 g (0,04 mole) du produit attendu, pratiquement pur.

CCM ($CH_3OH$) : Rf = 0,2

RMN$^{13}$C : δ(ppm) : 171,6 (C=O) ; 149,2 (C-CO) ; 110,8 (C-I) ; 73-69 (CHOH); 65-62 ($CH_2OH$) ; 54-50 ($CH_2N$).

EXEMPLE 7

N,N'-bis[(2,3-dihydroxypropyl) (2,3,4,5,6-pentahydroxyhexyl)]-2,3,5,6-tétraiodo-téréphtalamide

(formule II : $R_7 = R'_7 = CH_2\text{-}CHOH\text{-}CH_2OH$ ;

$R_8 = R'_8 = CH_2\text{-}(CHOH)_4\text{-}CH_2OH$)

66,4 g (0,26 mole) de 5-(2,3-dihydroxypropylamino) 1,3,4,5,6-hexanepentol, préparé comme décrit dans US 2,258,834, en solution dans 560 ml de DMAC, sont introduits, goutte à goutte, à 60° C, dans une solution dans 560 ml de DMAC de 61,3 g (0,087 mole) du dichlorure de l'acide 2,3,5,6-tétraiodotéraphtalique et 26,3 g (0,26 mole) de triéthylamine.

Après 3 jours d'agitation à 60° C, on concentre sous pression réduite. L'huile résiduelle est passée sur résines IRN 77 et IRA 67, puis purifiée par chromatographie préparative comme à l'exemple précédent.

On isole ainsi 32,1 g (0,028 mole) du composé attendu pratiquement pur.

CCM ($CH_3OH/CH_2Cl_2$ : 60/40 : v/v) : Rf = 0,16

RMN$^{13}$C δ(ppm) : 171,6 (C=O) ; 148,6 ( C-CO) ; 111 (C-I); 73-69 (CHOH) ; 63 ($CH_2OH$) ; 54-51 ($CH_2$-N).

EXEMPLE 8

N,N'-tétrakis(2,3,4,5,6-pentahydroxyhexyl)-2,3,5,6-tétraiodotéraphtalamide

$$(\text{formule II}: R_7 = R'_7 = R_8 = R'_8 = CH_2\text{-}(CHOH)_4\text{-}CH_2OH)$$

58,7 g (0,17 mole) de disorbitylamine commerciale en solution dans 750 ml de DMAC sont introduits, goutte à goutte, à 65° C, dans une solution de 31,2 g (0,04 mole) du dichlorure d'acide, de 17,2 g (0,17 mole) de triéthylamine et de 750 ml de DMAC. Après 3 jours d'agitation à 70° C et une nuit à température ambiante, le milieu réactionnel est concentré sous pression réduite et l'huile résiduelle passée sur résines IRN 77 et IRA 67 puis chromatographiée dans un appareil de chromatographie haute pression préparative, dans les mêmes conditions qu'à l'exemple 6.

On obtient ainsi 11,6 g (8,8 mmole) du produit attendu pratiquement pur.

CCM ($CH_3OH$) : Rf = 0,15

RMN$^{13}$C δ(ppm) : 172 (C=O); 149 (C-C=O); 111,4 (C-I) ; 71,4 (CHOH) ; 63,3 ($CH_2OH$) ; 54-50 ($CH_2N$).

EXEMPLE 9

N,N'-tétrakis(2,3,4,5-tétrahydroxypentyl)-2,3,5,6-tétraiodotéréphtalamide

$$(\text{formule II}: R_7 = R'_7 = R_8 = R'_8 = CH_2\text{-}(CHOH)_3\text{-}CH_2OH)$$

En suivant le mode opératoire de l'exemple 10 à partir de 1,3 g de dichlorure d'acide et 1,7 g de l'aminoalcool approprié, mais en ne laissant le milieu réactionnel que 24 heures à 50° C après la fin de l'addition, on obtient 800 mg du produit attendu.

CCM ($CH_3OH/CH_2Cl_2$ : 70/30 : v/v) : Rf = 0,25

RMN$^{13}$C δ(ppm) : 171 (C=O) ; 149 (C-C=O) 110 (CI); 73-69 (CHOH) ; 62 ($CH_2OH$) ; 54-50 ($CH_2N$).

EXEMPLE 10

N,N'-bis[(2,3,4-trihydroxybutyl)(2,3,4,5-tétrahydroxypentyl)]-2,3,5,6-tétraiodotéraphtalamide

$$(\text{formule II } R_7 = R'_7 = CH_2\text{-}(CHOH)_2\text{-}CH_2OH$$

$$R_8 = R'_8 = CH_2\text{-}(CHOH)_3\text{-}CH_2OH)$$

En appliquant la méthode décrite à l'exemple 9 à 1,4 g de dichlorure d'acide et 3,9 g de l'amino-alcool approprié, on obtient 700 mg du produit attendu pratiquement pur.

CCM ($CH_3OH/CH_2Cl_2$ : 60/40 : v/v) : Rf = 0,15

RMN$^{13}$C δ(ppm ) : 171 (C=O) ; 148 ($\underline{C}$-CO) ; 110 (C-I); 73-68 (CHOH); 62 ($CH_2OH$) ; 54-50 ($NCH_2$).

**Revendications**

**1.** Composés de formule :

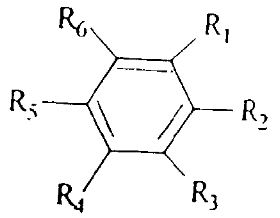

(I)

dans laquelle $R_1$, $R_2$, $R_3$, $R_4$, $R_5$, $R_6$ sont choisis indépendamment parmi l'atome d'iode et les groupes

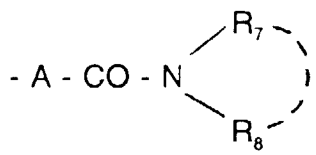

;

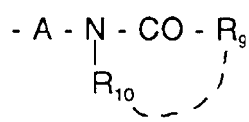

et

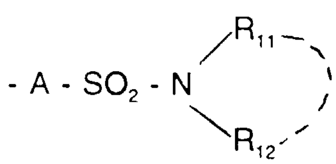

dans lesquels :

A représente rien ou $(C_1-C_4)$alkylène, éventuellement hydroxylé ; $R_7$, $R_8$, $R_9$, $R_{10}$, $R_{11}$ et $R_{12}$, identiques ou différents, représentent H, $(C_1-C_{10})$alkyle éventuellement hydroxylé ou portant un ou des groupes $(C_1-C_3)$alkoxy éventuellement hydroxylés, ou avec l'atome d'azote auquel ils sont attachés, $R_7$ et $R_8$, $R_9$ et $R_{10}$, $R_{11}$ et $R_{12}$ peuvent former indépendamment un hétérocycle saturé de 5 à 8 sommets, éventuellement hydroxylé,

étant entendu

qu'au moins 4 groupes parmi $R_1$, $R_2$, $R_3$, $R_4$, $R_5$ et $R_6$ représentent l'atome d'iode et que les composés de formule I contiennent de 10 à 24 groupes hydroxyles,

les alkyle, alkoxy et alkylène pouvant être linéaires ou ramifiés.

**2.** Composés selon la revendication 1 de formule (I), caractérisés en ce qu'ils sont tétraiodés et que les deux autres substituants représentent indépendamment

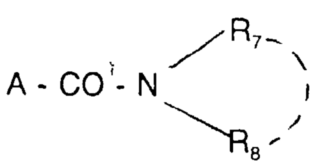

ou

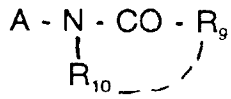

A étant $CH_2$ ou rien, $R_7$, $R_8$, $R_9$ et $R_{10}$ étant tels que définis à la revendication 1.

**3.** Composés selon la revendication 1 de formule (I), dans laquelle A n'est rien, $R_2 = R_3 = R_5 = R_6 = I$ et $R_1$ et $R_4$ représentent indépendamment un groupe $CO\text{-}NR_7R_8$ hydroxylé ou un groupe $N(R_{10})\text{-}CO\text{-}R_9$ hydroxylé, lesdits composés comportant de 12 à 20 hydroxyles.

**4.** Composés selon la revendication 1, comportant de 12 à 16 hydroxyles de formule I dans laquelle $R_2 = R_3 = R_5 = R_6 = I$; $R_1$ et $R_4$ représentent indépendamment $CO\text{-}NR_7R_8$ ou $N(R_{10})\text{-}CO\text{-}R_9$ dans lesquels $R_7$, $R_8$, $R_9$ et $R_{10}$ représentent indépendamment l'un de l'autre,
$-(CH_2)_m-(CHOH)_n-CH_2OH$ et m = 1 ou 2, n = 0 à 4 ou

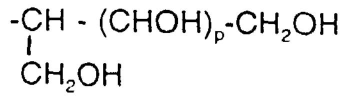

et p = 0 à 3
ou $-C(CH_2OH)_3$.

**5.** Composés selon la revendication 1, de formule II :

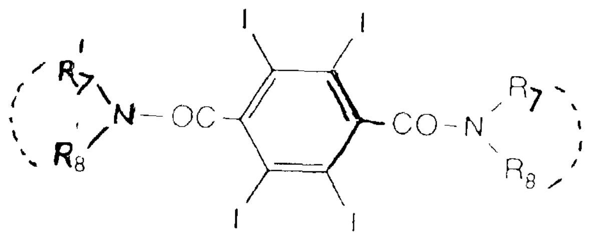

(II)

dans laquelle $R_7$, $R_8$, $R'_7$, $R'_8$, identiques ou différents, ont les significations de $R_7$ et $R_8$ de la formule I.

**6.** Composés selon la revendication 5 de formule II, dans laquelle les deux groupes amides sont différents et chacun comporte au moins deux hydroxyles.

**7.** Composés selon la revendication 5 de formule II, dans laquelle les deux groupes amides sont identiques.

**8.** Composés selon la revendication 5, comportant de 12 à 20 groupes hydroxyle, de formule II, dans laquelle les deux groupes amides sont identiques et $R_7$, $R_8$, $R'_7$ et $R'_8$ sont choisis parmi
$-(CH_2)_m-(CHOH)_n-CH_2OH$ avec m = 1 ou 2, n = 0 à 4,

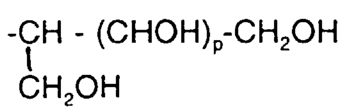

avec p = 0 à 3,
ou $-C(CH_2OH)_3$.

**9.** Composés selon la revendication 5 comportant de 12 à 20 groupes hydroxyle, de formule II, dans laquelle $NR_7R_8$ et $NR'_7R'_8$ sont identiques et $R_7$, $R_8$, $R'_7$, $R'_8$ sont choisis parmi les groupes
$CH_2-(CHOH)_n-CH_2OH$ avec n = 0 à 4, et

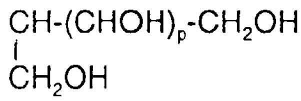

avec p = 1 ou 3.

**10.** Composés selon la revendication 5 de formule II dans laquelle $NR_7R_8$ et $NR'_7R'_8$ sont identiques et $R_7$, $R_8$, $R'_7$, $R'_8$ sont choisis parmi les groupes $CH_2$-$(CHOH)_n$-$CH_2OH$ avec n = 0 à 3, lesdits composés de formule II comportant de 12 à 16 hydroxyles.

**11.** Composé selon la revendication 5 de formule II, dans laquelle $NR_7R_8$ et $NR'_7R'_8$ représentent :

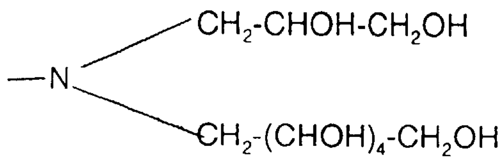

**12.** Composé selon la revendication 5 de formulé II, dans laquelle $NR_7R_8$ et $NR'_7R'_8$ représentent :

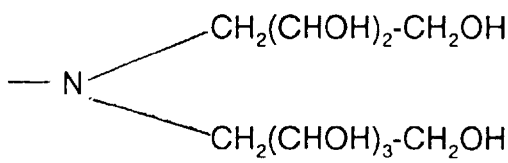

**13.** Procédé de préparation des composés de formule I dont les substituants sont choisis parmi l'atome d'iode et les groupes

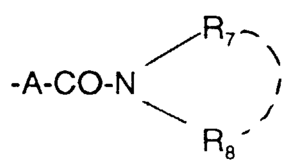

où $R_7$ et $R_8$, identiques ou différents, ont la même signification qu'à la revendication 1, et A est tel que défini à la revendication 1, comprenant l'étape consistant à faire réagir un dérivé activé du composé acide carboxylique correspondant sur l'aminoalcool approprié.

**14.** Procédé de préparation des composés tétraiodés de formule I dans laquelle

$$R_2 = R_3 = R_5 = R_6 = I;$$

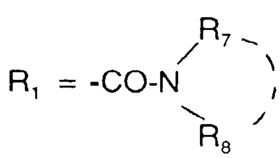 ;

et

$R_4$ = 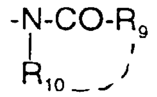

où $R_7$, $R_8$, $R_9$ et $R_{10}$, identiques ou différents, ont la même signification qu'à la revendication 1, comprenant les étapes consistant à :

1/ faire une iodation du p-nitrotoluène pour obtenir le 2,6-diiodo-4-nitrotoluène ;
2/ faire réagir le N-bromosuccinimide pour obtenir le composé de formule

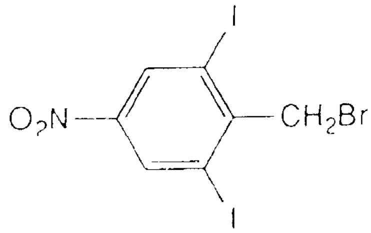

3/ oxyder ce dérivé bromé pour obtenir la 2,6-diiodo-4-nitrobenzaldéhyde puis l'acide 2,6-diiodo-4-nitrobenzoïque;
4/ faire réagir un dérivé activé de l'acide obtenu à l'étape précédente sur l'aminoalcool $HNR_7R_8$;
5/ effectuer la réduction catalytique du groupe nitro en amino ;
6/ procéder à l'iodation de l'aniline obtenue à l'étape précédente ;
7/ faire réagir un dérivé activé de l'acide $R_9$-COOH sur le composé iodé résultant, les fonctions hydroxyles étant éventuellement protégées;
8/ faire réagir un composé de formule $R_{10}$-X" dans laquelle X" représente un halogène, un groupe ($C_1$-$C_4$) alkylsulfonyle ou ($C_6$-$C_{10}$)arylsulfonyle pour obtenir l'amide tertiaire;
9/ éventuellement déprotéger les hydroxyles.

**15.** Composition de diagnostic pour radiographie par rayons X contenant comme principe actif un composé selon l'une des revendications 1 à 12, associé à un véhicule pharmaceutiquement acceptable.

**16.** Composition de diagnostic selon la revendication 15, caractérisée en ce qu'elle contient comme principe actif un composé de formule :

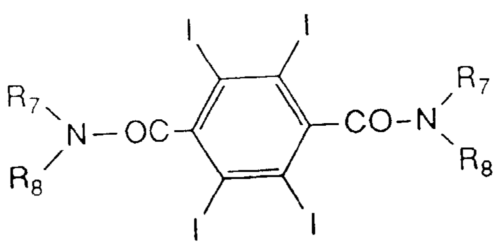

dans laquelle $R_7$ et $R_8$ sont choisis indépendamment parmi les groupes $-CH_2-(CHOH)_n-CH_2OH$ avec n 0 à 4 et

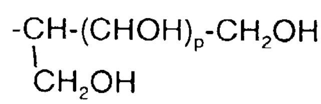

avec p = 1 ou 3.

**17.** Composition de diagnostic selon la revendication 15, caractérisée en ce qu'elle contient comme principe actif un composé de formule :

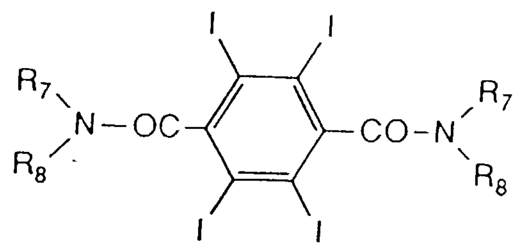

dans laquelle $R_7$ et $R_8$ sont choisis indépendamment parmi les groupes $-CH_2-(CHOH)_n-CH_2OH$ avec n = 0 à 3, qui contient de 12 à 16 hydroxyles.

**18.** Aminoalcool de formule :

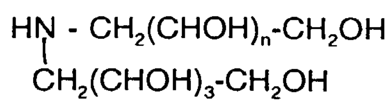

dans laquelle n = 1 à 3,

**19.** Aminoalcools de formule :

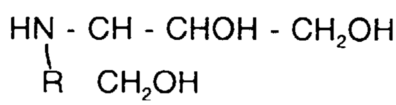

dans laquelle $R = CH_2(CHOH)_p-CH_2OH$ avec p = 2,3.

**20.** Aminoalcools de formule :

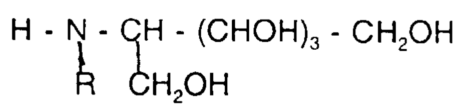

dans laquelle $R = CH_2-(CHOH)_p-CH_2OH$ avec p = 1 à 3.

**Patentansprüche**

**1.** Verbindungen der Formel

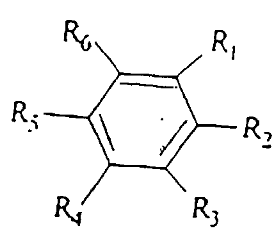

(I)

wobei $R_1$, $R_2$, $R_3$, $R_4$, $R_5$, $R_6$ unabhängig voneinander aus einem Iodatom und den Gruppen

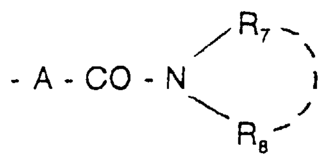

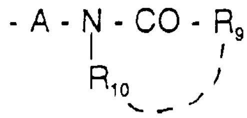

und

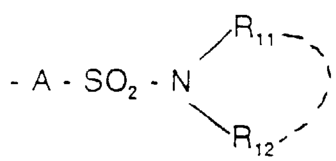

ausgewählt werden, in denen:

A nichts oder gegebenenfalls hydroxyliertes ($C_1$-$C_4$)Alkylen darstellt;
$R_7$, $R_8$, $R_9$, $R_{10}$, $R_{11}$ und $R_{12}$, gleich oder verschieden, H, ($C_1$-$C_{10}$)Alkyl darstellen, gegebenenfalls hydroxyliert oder gegebenenfalls hydroxylierte ($C_1$-$C_3$)Alkoxygruppen tragend, oder $R_7$ und $R_8$, $R_9$ und $R_{10}$, $R_{11}$ und $R_{12}$ gemeinsam mit dem Stickstoffatom, an das sie gebunden sind, unabhängig voneinander einen gesättigten Heterocyclus mit 5 bis 8 Atomen, gegebenenfalls hydroxyliert, bilden können,
vorausgesetzt, daß
mindestens 4 Gruppen von $R_1$, $R_2$, $R_3$, $R_4$, $R_5$ und $R_6$ ein Iodatom darstellen und daß die Verbindungen der Formel I 10 bis 24 Hydroxylgruppen enthalten, und daß Alkyl, Alkoxy und Alkylen linear oder verzweigt sein können.

**2.** Verbindungen nach Anspruch 1 der Formel (I), dadurch charakterisiert, daß sie tetraiodiert sind und daß die anderen Substituenten unabhängig voneinander

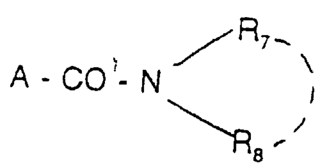

oder

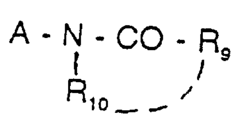

darstellen, wobei A $CH_2$ oder nichts darstellt, und $R_7$, $R_8$, $R_9$ und $R_{10}$ so wie in Anspruch 1 definiert sind.

**3.** Verbindungen nach Anspruch 1 der Formel (I), wobei A nichts darstellt, $R_2 = R_3 = R_5 = R_6 = I$ und $R_1$ und $R_4$ unabhängig voneinander eine hydroxylierte $CO-NR_7R_8$-Gruppe oder eine hydroxylierte $N(R_{10})-CO-R_9$-Gruppe darstellen, woebi die Verbindungen 12 bis 20 Hydroxyl umfassen.

**4.** Verbindungen nach Anspruch 1 der Formel I, umfassend 12 bis 16 Hydroxyl, wobei $R_2 = R_3 = R_5 = R_6 = I$; $R_1$ und $R_4$ unabhängig voneinander $CO\text{-}NR_7R_8$ oder $N(R_{10})\text{-}CO\text{-}R_9$ darstellen, wobei $R_7$, $R_8$, $R_9$ und $R_{10}$ unabhängig voneinander $-(CH_2)_m-(CHOH)_n-CH_2OH$ und m = 1 oder 2, n = 0 bis 4 oder

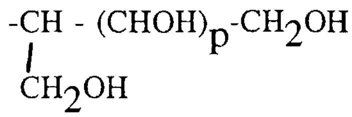

und p = 0 bis 3 oder $-C(CH_2OH)_3$ darstellen.

**5.** Verbindungen nach Anspruch 1 der Formel II

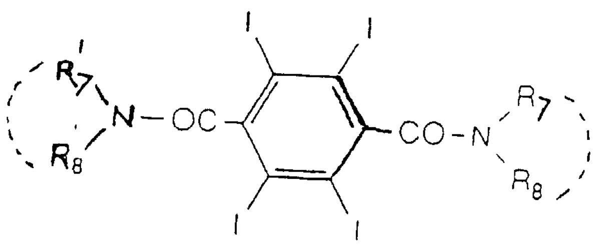

(II)

wobei $R_7$, $R_8$, $R'_7$, $R'_8$ gleich oder verschieden, die Bedeutungen von $R_7$ und $R_8$ der Formel I aufweisen.

**6.** Verbindungen der Formel II nach Anspruch 5, wobei die beiden Amidgruppen verschieden sind und jede mindestens zwei Hydroxyl trägt.

**7.** Verbindungen der Formel II nach Anspruch 5, wobei die beiden Amidgruppen identisch sind.

**8.** Verbindungen der Formel II nach Anspruch 5, umfassend 12 bis 20 Hydroxylgruppen, wobei beide Amidgruppen identisch sind und $R_7$, $R_8$, $R'_7$ und $R'_8$ ausgewählt werden unter den Gruppen $-(CH_2)_m-(CHOH)_n-CH_2OH$ und m = 1 oder 2, n = 0 bis 4 oder

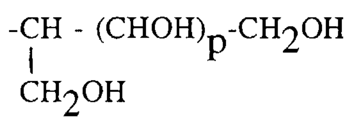

und p = 0 bis 3 oder $-C(CH_2OH)_3$.

**9.** Verbindungen der Formel II nach Anspruch 5, umfassend 12 bis 20 Hydroxylgruppen, wobei $NR_7R_8$ und $NR'_7R'_8$ identisch sind und $R_7$, $R_8$, $R'_7$ und $R'_8$ ausgewählt werden unter den Gruppen $-(CH_2)_m-(CHOH)_n-CH_2OH$ mit n = 0 bis 4 oder

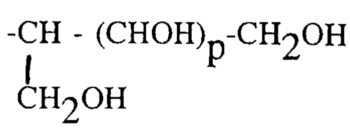

und p = 0 bis 3.

**10.** Verbindungen der Formel II nach Anspruch 5, wobei $NR_7R_8$ und $NR_7'R_8'$ identisch sind und $R_7$, $R_8$, $R_7'$, $R_8'$ unter den Gruppen $CH_2$-$(CHOH)_n$-$CH_2OH$ mit n = 0 bis 3 ausgewählt werden, wobei die Verbindungen der Formel II 12 bis 16 Hydroxyl umfassen.

**11.** Verbindungen der Formel II nach Anspruch 5, wobei $NR_7R_8$ und $NR_7'R_8'$ darstellen:

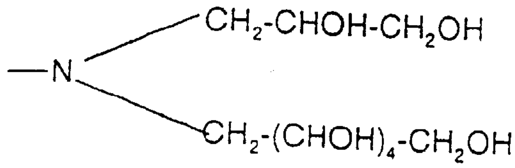

**12.** Verbindungen der Formel II nach Anspruch 5, wobei $NR_7R_8$ und $NR_7'R_8'$ darstellen:

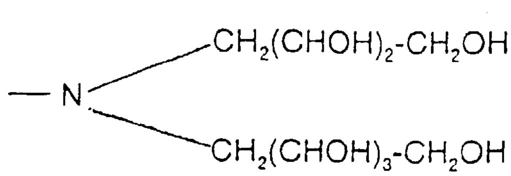

**13.** Verfahren zur Herstellung von Verbindungen der Formel I, wobei die Substituenten ausgewählt werden unter einem Iodatom und den Gruppen

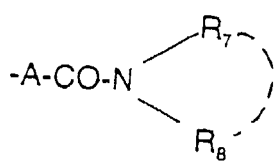

wobei $R_7$ und $R_8$, gleich oder verschieden, die gleiche Bedeutung haben wie in Anspruch 1 und A wie in Anspruch 1 definiert ist, umfassend einen Schritt, der aus der Reaktion eines aktivierten Derivates der entsprechenden Carbonsäureverbindung mit dem geeigneten Aminoalkohol besteht.

**14.** Verfahren zur Herstellung von tetraiodierten Verbindungen der Formel I, wobei

$$R_2 = R_3 = R_5 = R_6 = I;$$

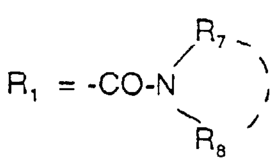 ;

und

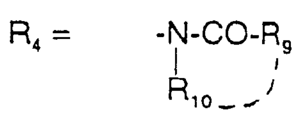

wobei $R_7$, $R_8$, $R_9$ und $R_{10}$, gleich oder verschieden, dieselbe Bedeutung haben wie in Anspruch 1, umfassend die Schritte, die bestehen aus:

1) Iodierung von p-Nitrotoluol zur Herstellung von 2,6-Diiodo-4-nitrotoluol;
2) Reaktion von N-Bromosuccinimid zur Herstellung der Verbindung der Formel

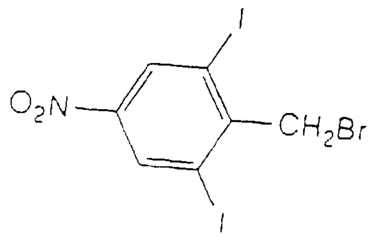

3) Oxidation dieses bromierten Derivats zur Herstellung von 2,6-Diiodo-4-nitrobenzaldehyd und anschließend 2,6-Diiodo-4-nitrobenzoesäure;
4) Reaktion eines aktivierten Derivats der im vorstehenden Schritt erhaltenen Säure mit dem Aminoalkohol $HNR_7R_8$;
5) Durchführung der katalytischen Reduktion der Nitrogruppe zur Aminogruppe;
6) Durchführung der Iodierung des im vorstehenden Schritt erhaltenen Anilins;
7) Reaktion eines aktivierten Derivats der Säure $R_9$-COOH mit der resultierenden iodierten Verbindung, wobei die Hydroxylfunktionen gegebenenfalls geschützt sind;
8) Reaktion einer Verbindung der Formel $R_{10}$-X", wobei X" ein Halogen, eine ($C_1$-$C_4$)Alkylsulfonylgruppe oder ($C_6$-$C_{10}$)Arylsulfonylgruppe darstellt, zur Herstellung des tertiären Amids;
9) gegebenenfalls Entschützung der Hydroxylgruppen.

**15.** Diagnostische Zusammensetzungen für die Radiographie durch Röntgenstrahlen, enthaltend als aktiven Wirkstoff eine Verbindung nach einem der Ansprüche 1 bis 12 in Verbindung mit einem pharmazeutisch verträglichen Vehikel.

**16.** Diagnostische Zusammensetzung nach Anspruch 15, dadurch charakterisiert, daß sie als aktiven Wirkstoff eine Verbindung der Formel

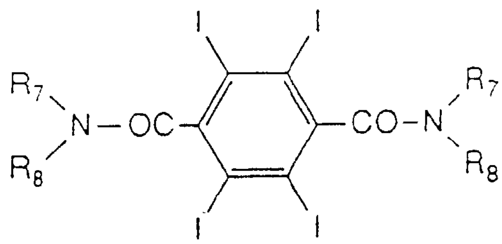

wobei $R_7$ und $R_8$ unabhängig voneinander unter den Gruppen $-(CH_2)_m-(CHOH)_n-CH_2OH$ mit n = 0 bis 4 und

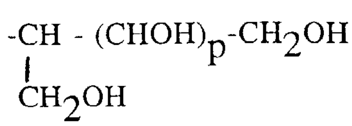

mit p = 0 oder 3 ausgewählt werden.

**17.** Diagnostische Zusammensetzung nach Anspruch 15, dadurch charakterisiert, daß sie als aktiven Wirkstoff eine

Verbindung der Formel

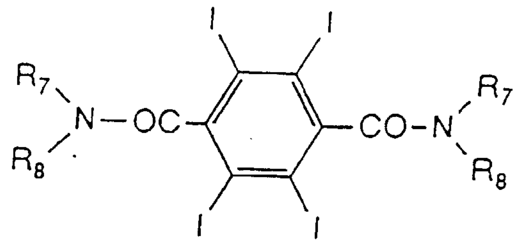

enthält, wobei $R_7$ und $R_8$ unabhängig voneinander unter den Gruppen $-CH_2-(CHOH)_n-CH_2OH$ mit n = 0 bis 3 ausgewählt werden, und die 12 bis 16 Hydroxyl enthält.

**18.** Aminoalkohol der Formel:

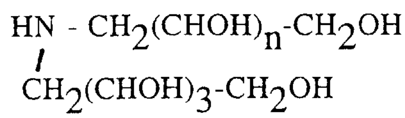

wobei n = 1 bis 3 ist.

**19.** Aminoalkohole der Formel:

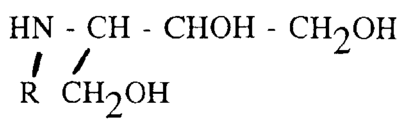

wobei $R = CH_2(CHOH)_p-CH_2OH$ mit p = 2,3 ist.

**20.** Aminoalkohole der Formel:

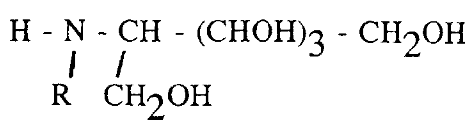

wobei $R = CH_2-(CHOH)_p-CH_2OH$ mit P = 1 bis 3 ist.

**Claims**

**1.** Compounds of the formula:

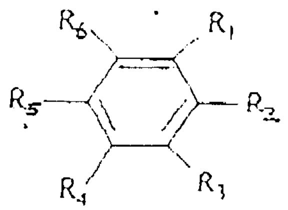

(I)

wherein $R_1$, $R_2$, $R_3$, $R_4$, $R_5$, $R_6$ are selected independently from iodine and the groups

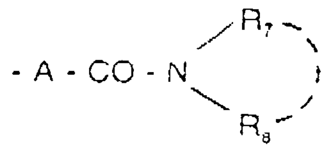

;

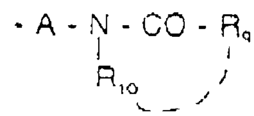

and

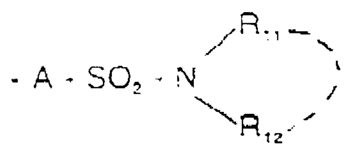

wherein:

A is absent or represents $(C_1-C_4)$alkylene which is optionally hydroxylated,
$R_7$, $R_8$, $R_9$, $R_{10}$, $R_{11}$ and $R_{12}$, which may be identical or different, represent H, $(C_1-C_{10})$alkyl which is optionally hydroxylated or which optionally carries one or more optionally hydroxylated $(C_1-C_3)$alkoxy groups, or, together with the nitrogen atom to which they are attached, $R_7$ and $R_8$, $R_9$, and $R_{10}$, $R_{11}$ and $R_{12}$ may independently form an optionally hydroxylated saturated heterocycle having from 5 to 8 ring members, with the proviso that at least 4 groups from among $R_1$, $R_2$, $R_3$, $R_4$, $R_5$ and $R_6$ represent iodine and that the compounds of formula (I) contain from 10 to 24 hydroxyl groups,
it being possible for the alkyl, alkoxy and alkylene groups to be linear or branched.

**2.** Compounds according to claim 1 of formula (I), characterised in that they are tetraiodised and in that the other two substituents represent independently

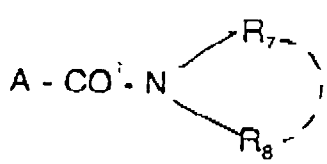

.

or

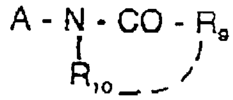

A being $CH_2$ or bring absent, $R_7$, $R_8$, $R_9$ and $R_{10}$ being as defined in claim 1.

**3.** Compounds according to claim 1 of formula (I), wherein A is absent, $R_2 = R_3 = R_5 = R_6 = I$ and $R_1$ and $R_4$ represent independently a hydroxylated $CO\text{-}NR_7R_8$ group or a hydroxylated $N(R_{10})\text{-}CO\text{-}R_9$ group, said compounds comprising from 12 to 20 hydroxyl groups.

**4.** Compounds according to claim 1, comprising from 12 to 16 hydroxyl groups, of formula (I), wherein $R_2 = R_3 = R_5 = R_6 = I$; $R_1$ and $R_4$ represent independently $CO\text{-}NR_7R_8$ or $N(R_{10})\text{-}CO\text{-}R_2$ wherein $R_7$, $R_8$, $R_9$ and $R_{10}$ represent, independently of one another,
$-(CH_2)_m\text{-}(CHOH)_n\text{-}CH_2OH$ and m = 1 or 2, n = 0 to 4 or

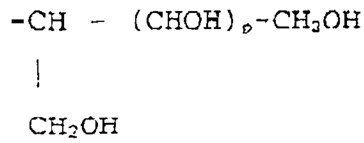

and p = 0 to 3
or $-C(CH_2OH)_3$.

**5.** Compounds according to claim 1, of formula (II) :

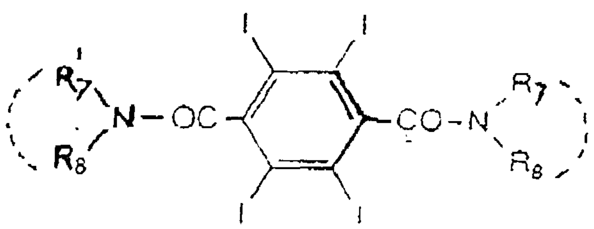

(II)

wherein $R_7$, $R_8$, $R'_7$, $R'_8$, which may be identical or different, have the meanings of $R_7$ and $R_8$ in formula (I).

**6.** Compounds according to claim 5 of formula (II), wherein the two amide groups are different and each comprises at least two hydroxyl groups.

**7.** Compounds according to claim 5 of formula (II), wherein the two amide groups are identical.

**8.** Compounds according to claim 5, comprising from 12 to 20 hydroxyl groups, of formula (II), wherein the two amide groups are identical and $R_7$, $R_8$, $R'_7$ and $R'_8$ are selected from
$-(CH_2)_m\text{-}(CHOH)_n\text{-}CH_2OH$ where m = 1 or 2, n = 0 to 4,

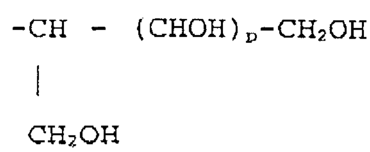

where p = 0 to 3,
or $-C(CH_2OH)_3$.

**9.** Compounds according to claim 5, comprising from 12 to 20 hydroxyl groups, of formula (II), wherein $NR_7R_8$ and $NR'_7R'_8$ are identical and $R_7$, $R_8$, $R'_7$, $R'_8$ are selected from the groups $CH_2\text{-}(CHOH)_n\text{-}CH_2OH$ where n = 0 to 4, and

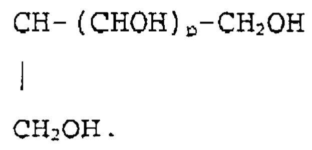

where p = 1 or 3.

**10.** Compounds according to claim 5 of formula (II), wherein $NR_7R_8$ and $NR'_7R'_8$ are identical and $R_7$, $R_8$, $R'_7$, R'8 are selected from the groups $CH_2\text{-}(CHOH)_n\text{-}CH_2OH$ where n = 0 to 3, the compounds of formula (II) comprising from 12 to 16 hydroxyl groups.

**11.** Compound according to claim 5 of formula (II), wherein $NR_7R_8$ and $NR'_7R'_8$ represent;

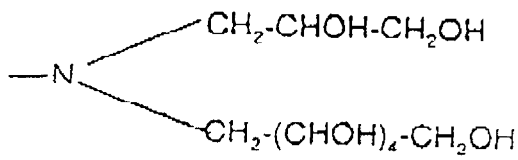

**12.** Compound according to claim 5 of formula (II), wherein $NR_7R_8$ and $NR'_7R'_8$ represent:

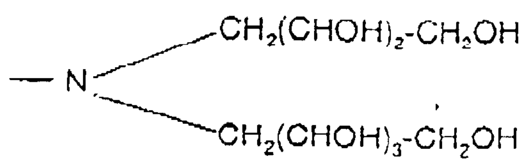

**13.** Process for the preparation of the compounds of formula (I), of which the substituents are selected from iodine and the groups

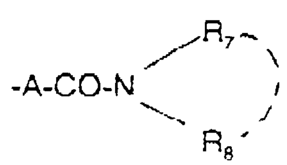

wherein $R_7$ and $R_8$, which may be identical or different, have the same meaning as in claim 1, and A is as defined in claim 1,
comprising the step which consists in reacting an activated derivative of the corresponding carboxylic acid compound with the appropriate aminoalcohol.

**14.** Process for the preparation of the tetraiodised compounds of formula (I) wherein

$$R_2 = R_3 = R_5 = R_6 = I,$$

$R_1$ = 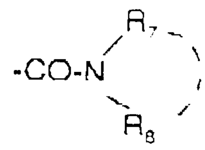 ;

and

$R_4$ = 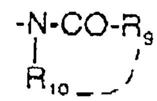

wherein $R_7$, $R_8$, $R_9$ and $R_{10}$, which may be identical or different, have the same meaning as in claim 1, comprising the steps which consist in:

1/ iodising p-nitrotoluene to obtain 2,6-diiodo-4-nitrotoluene;
2/ reacting N-bromosuccinimide to obtain the compound of the formula

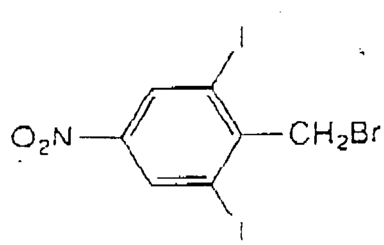

3/ oxidising that brominated derivative to obtain 2,6-diiodo-4-nitrobenzaldehyde and then 2,6-diiodo-4-nitrobenzoic acid;
4/ reacting an activated derivative of the acid obtained in the previous step with the aminoalcohol $HNR_7R_8$;
5/ effecting the catalytic reduction of the nitro group to amino;
6/ iodising the aniline obtained in the previous step;
7/ reacting an activated derivative of the acid $R_4$-COOH with the resulting iodised compound, the hydroxyl functions optionally being protected;
8/ reacting a compound of the formula $R_{10}$-X" wherein X" represents a halogen, a ($C_1$-$C_4$)alkylsulphonyl or ($C_6$-$C_{10}$)-arylsulphonyl group to obtain the tertiary amide;
9/ optionally deprotecting the hydroxyl groups.

**15.** Diagnostic composition for x-ray radiography, containing as active ingredient a compound according to any one of claims 1 to 12, associatad with a pharmaceutically acceptable carrier.

**16.** Diagnostic composition according to claim 15, characterised in that it contains as active ingredient a compound of the formula:

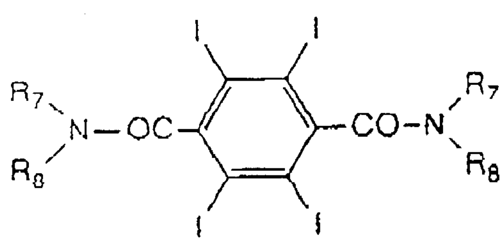

wherein $R_7$ and $R_8$ are selected independently from the groups

$-CH_2-(CHOH)_n-CH_2OH$ where n = 0 to 4, and

$-CH-(CHOH)_p-CH_2OH$
|
$CH_2OH$.

where p = 1 or 3.

**17.** Diagnostic composition according to claim 15, characterised in that it contains as active ingredient a compound of the formula:

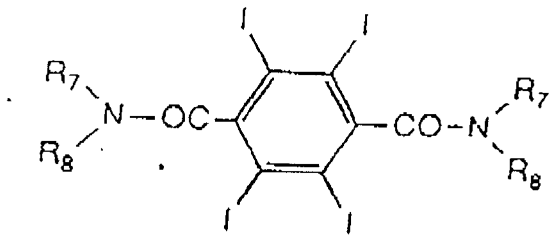

wherein $R_7$ and $R_8$ are selected independently from the groups $-CH_2-(CHOH)_n-CH_2OH$ where n = 0 to 3, which contains from 12 to 16 hydroxyl groups.

**18.** Aminoalcohol of the formula:

$HN - CH_2(CHOH)_n-CH_2OH$
|
$CH_2(CHOH)_3-CH_2OH$

wherein n = 1 to 3.

**19.** Aminoalcohols of the formula:

$HN - CH - CHOH - CH_2OH$
| |
R $CH_2OH$

wherein $R = CH_2(CHOH)_p-CH_2OH$ where p = 2, 3.

**20.** Aminoalcohols of the formula:

$H - N - CH - (CHOH)_3 - CH_2OH$
| |
R $CH_2OH$

wherein R - $CH_2$-$(CHOH)_p$-$CH_2OH$ where p = 1 to 3.